# EUROPÄISCHE PATENTANMELDUNG

(11) **EP 2 071 482 A1**
(43) Veröffentlichungstag der Anmeldung: **17.06.2009**
(21) Anmeldenummer: 08021514.8
(22) Anmeldetag: 11.12.2008
(51) Int. Cl.: G06F 19/00

(54) **Verfahren und Identifikatorsystem zur Zuordnung von Fotodokumenten zu einem Patienten**

(30) Priorität: 11.12.2007 DE 102007059973
(71) Anmelder: Medical Laser Rent E.K., 46485 Wesel (DE)
(72) Erfinder: Fraatz, Jürgen, 46485 Wesel (DE)
(74) Vertreter: COHAUSZ DAWIDOWICZ HANNIG & SOZIEN

(57) **Zusammenfassung**

Die Erfindung betrifft ein Verfahren zur Zuordnung von Bilddokumenten zu einem Patienten, bei dem wenigstens ein bildlich aufzunehmendes Areal (1a) wenigstens eines Patienten (1) mit wenigstens einem Identifikator (3a) gekennzeichnet wird und wobei jedes aufzunehmende Areal (1a) wenigstens einen Identifikator (3a) beinhaltet, der einem Patienten (1) zugeordnet ist oder in einem späteren Schritt zugeordnet wird und bei dem mit wenigstens einem Identifikator (3a) gekennzeichnete Areale (1a) jeweils zusammen mit dem wenigstens einen Identifikator (3a) als Bild oder Film, insbesondere Digitalfoto oder Digitalvideo erfasst werden oder in das Bild eines Areals (1a) nachträglich wenigstens ein Identifikator (3a) eingefügt wird und bei dem jedes Bild digitale Daten darstellt oder in digitale Daten gewandelt wird und bei dem die digitalen Daten wenigstens eines Bildes, insbesondere eines Datensatzes von mehreren Bildern, insbesondere verschiedener Patienten (1) untersucht werden auf das Vorhandensein wenigstens eines Identifikators (3a) und ein vorhandener Identifikator (3a) aus den digitalen Daten ausgelesen wird und anhand des so gewonnenen wenigstens einen Identifikators das Bild und/oder die digitalen Daten der Bilder dem durch den Identifikator identifizierten Patienten (1) zugeordnet wird. Die Erfindung betrifft weiterhin auch ein Identifikatorsystem zur Zuordnung von Fotodokumenten zu einem Patienten, welches einen Satz (2) von mehreren Identifikatoren (3a) aufweist, mit denen ein fotografisch oder videografisch aufzunehmendes Areal (1a) eines Patienten (1) kennzeichenbar ist, insbesondere durch Anbringung eines Identifikators (3a), wobei jeder Identifikator (3a) mit wenigstens einer optischen Information (4) versehen oder versehbar ist, die zumindest einem Patienten (1)zugeordnet ist oder zuordenbar ist.

## Beschreibung

Die Erfindung betrifft ein Verfahren zur Zuordnung von Bilddokumenten zu einem Patienten bzw. Kunden. Die Erfindung betrifft weiterhin auch ein Identifikatorsystem, um eine solche Zuordnung durchzuführen.

Im Stand der Technik ist es allgemein bekannt, Bilddokumente, z.B. Fotodokumente von Patienten bzw. Kunden, d.h. hier im Bereich der Medizin und/oder Kosmetik, z.B. EDV-gestützt systematisch abzulegen. Beispielsweise auf dem Gebiet der Dermatologie werden von einem Arzt häufig Hautveränderungen fotografisch festgehalten, um über eine längere Zeit beobachten zu können, wie sich eine Hautveränderung entwickelt. So kann eine solche Hautveränderung zu einem bestimmten Zeitpunkt verglichen werden mit dem Fotodokument derselben Hautveränderung aus der Vergangenheit.

Dabei ist es als ein Verfahren bekannt, dass ein Arzt auf seinem Computer mit einer Patientendatenbank den Patientendatensatz des gerade zu untersuchenden Patienten öffnet und mittels einer an den Computer direkt angeschlossenen Kamera, z.B. über einen USB-Anschluss verbundenen Kamera, ein zu fotografierendes Areal des Patienten fotografisch erfasst und das so aufgenommene Bild unmittelbar in den geöffneten Patientendatensatz eingeschrieben wird und somit für spätere zukünftige Betrachtungen gespeichert ist, da ein solches Fotodokument aus dem Patientendatensatz immer wieder abrufbar ist, sofern es nicht explizit gelöscht wird.

Dabei ist es im Stand der Technik auch bekannt, dass der Arzt, der das Foto von einem Areal eines Patienten aufnimmt, zu jedem Foto unmittelbar vor oder nach der fotografischen Erfassung des Areals am Computer eine Angabe darüber macht, welches Areal des Patienten fotografiert wurde, also beispielsweise ein Areal an der Hand, am Fuß, am Oberkörper, Kopf oder einer sonstigen Extremität oder Lokalität des Patienten. Dies ist deshalb vorgesehen, da die fotografische Wiedergabe des fotografierten Areals eines Patienten üblicherweise nur einen kleinen Hautausschnitt zeigt und somit anhand des fotografierten Bildes nachträglich nicht mehr festgestellt werden kann, um welches Areal es sich handelt, so dass eine unmittelbare Zuordnung sowohl des Patienten als auch des fotografierten Areals am Computer in dem gerade geöffneten Patientendatensatz nötig ist.

Ein derart bekanntes Verfahren hat den Nachteil, dass die Durchführung des Verfahrens nur bei geöffnetem Patientendatensatz möglich ist, um so die zu einem Foto zugehörigen Informationen über die Identifikation des Patienten sowie das fotografierte Areal unmittelbar dem geöffneten Patientendatensatz bekannt zu geben. Dies bedeutet, dass die Durchführung einer Fotodokumentation nur sinnvoll möglich ist, wenn sich der Patient in den Praxisräumen des Arztes befindet, wo dieser über seinen Computer Zugriff auf seine Patientendatenbank und somit den entsprechenden Patientendatensatz des gerade zu untersuchenden Patienten Zugriff hat. Die Durchführung des Verfahrens ist in großen Krankenhäusern oder bei Hausbesuchen des Arztes nicht praktizierbar, es sei denn, dass der Arzt sich notiert, welches Bild zu welchem Patienten gehört und nachträglich zuordnet oder er die Computerinfrastruktur mitführt, was jeweils aufwendig ist, insbesondere da er ggfs. auch die gesamte Patientendatenbank mit sich führen muss.

Aufgabe der Erfindung ist es demnach, ein Verfahren zur Zuordnung von Bilddokumenten, wie z.B. Foto- oder Videodokumenten zu einem Patienten sowie auch ein Identifikatorsystem zur Zuordnung von Bilddokumenten zu einem Patienten bereitzustellen, welches die Erstellung der Bilddokumente vollständig losgelöst von jeglicher Infrastruktur, abgesehen von einer Kamera zur Aufnahme von Bildern, ermöglicht und dennoch sicherstellt, dass die Zuordnung der Bilddokumente auf unverwechselbare Art und Weise zu einem Patienten erfolgt.

So soll das erfindungsgemäße Verfahren sowie auch das erfindungsgemäße Identifikatorsystem die Möglichkeit erschließen, Bilddokumente zu einem Patienten anzufertigen, ohne dass der untersuchende Arzt neben einer Kamera und einem genannten erfindungsgemäßen Identifikatorsystem weitere Hilfsmittel oder Informationen, wie die eingangs genannten Computer und die darauf gespeicherte Patientendatenbank benötigt. Vielmehr kann es hier erfindungsgemäß vorgesehen sein, die aufgenommenen Bilddokumente nachträglich in die Patientendatenbank, d.h. in den jeweiligen Patientendatensatz des betreffenden Patienten einzufügen.

Erfindungsgemäß ist es dafür vorgesehen, dass wenigstens ein bildlich aufzunehmendes Areal wenigstens eines Patienten mit wenigstens einem Identifikator gekennzeichnet wird, wobei jedes aufzunehmende Areal wenigstens einen Identifikator aufweist, der einem Patienten zugeordnet ist oder in einem späteren Schritt zugeordnet wird und entweder mit wenigstens einem Identifikator versehene Areale jeweils zusammen mit dem wenigstens einen Identifikator als Bild/Bildsequenz, insbesondere Digitalfoto oder Digitalvideo erfasst werden, oder in das/die aufgenommene Bild/Bildsequenz eines Areals nachträglich wenigstens ein Identifikator, insbesondere als Bildinformation eingefügt wird, wobei jedes Bild, insbesondere bei Erfassung mit einer Digitalkamera, unmittelbar digitale Daten darstellt oder, insbesondere bei Aufnahme des Bildes/der Bildsequenz mit einer analogen Kamera, in digitale Daten gewandelt wird, wie z.B. durch nachträgliches Einscannen oder sonstige Art von Digitalisierung und wobei die digitalen Daten wenigstens eines Bildes, bevorzugt die digitalen Daten, d.h. ein Datensatz von mehreren Bildern und hier beispielsweise nicht nur von einem Patienten, sondern von verschiedenen Patienten untersucht werden auf das Vorhandensein wenigstens eines Identifikators, wobei ein vorhandener, d.h. während dieser Untersuchung festgestellter Identifikator, aus den digitalen Daten ausgelesen wird und anhand des so gewonnenen, wenigstens einen Identifikators das Bild und/oder die digitalen Daten des Bildes dem durch den Identifikator identifizierten Patienten zugeordnet wird. Insbesondere wird dabei unter dem Auslesen eines Identifikators aus den digitalen Daten verstanden, dass die durch einen solchen Identifikator repräsentierte wenigstens eine Information ausgelesen, d.h. zugänglich gemacht wird.

Gegenüber dem bekannten Stand der Technik hat ein erfindungsgemäßes Verfahren zur Zuordnung von Bilddokumenten zu einem Patienten somit als wesentliche vorteilhafte Merkmale, dass jede/s Bild oder Bildsequenz, welche/s von einem aufzunehmenden Areal eines beliebigen Patienten aufgenommen wird, erfindungsgemäß wenigstens einen Identifikator aufweist, der entweder vor der Aufnahme des Areals auf diesem Areal appliziert wurde z.B. durch physisches Anbringen oder während der Aufnahme von einer separaten oder einer Bilddokumentationseinheit inne wohnenden Apparatur projiziert wird oder der in das aufgenommene Bild nachträglich, insbesondere unmittelbar nach der Aufnahme als Bildinformation (z.B. Muster) in das Bild datentechnisch eingefügt wird, so dass jedes einzelne Bild oder eine Bildsequenz nachträglich und zwar hier zu einem beliebigen Zeitpunkt nach der Aufnahme, einem bestimmten Patienten zugeordnet werden kann, da hierfür lediglich der Identifikator aus dem Bildausgelesen werden muss, insbesondere was durch Bild- oder Mustererkennungsverfahren möglich ist.

So kann es in einer Ausführung vogesehen sein, dass eine Bilddokumentationseinheit (z.B. eine spezielle hierfür vorgesehene Kamera) nicht physische Identifikatoren enthält, welche entweder manuell oder automatisch innerhalb der aufgenommenen Bilder platziert werden.

Erfindungsgemäß kann es vorgesehen sein, dass ein solcher Identifikator, von dem wenigstens einer entweder an einem aufzunehmenden Areal eines Patienten physisch angebracht oder auf dieses projiziert oder nachträglich als Bildinformation in das Bild / eine Blldsequenz eingebracht wird , aus einem erfindungsgemäßen Identifikatorsystem stammt, welches einen Satz von mehreren Identifikatoren aufweist, mit denen ein fotografisch / videografisch aufzunehmendes Areal eines Patienten kennzeichenbar ist, wobei jeder Identifikator dieses Satzes mit wenigstens einer optischen Information versehen oder versehbar ist, die zumindest einem Patienten zugeordnet ist oder zuordenbar ist, also beispielsweise auch nachträglich zuordenbar ist.

Dabei kann die optische Information eines Identifikators, die zumindest zur Zuordnung zu einem Patienten dient, darin gesehen werden, dass ein Identifikator eine optische Information trägt, die entweder mit der fotografischen Aufnahme eines Bildes/Bildesequenz zusätzlich zum Areal mit aufgenommen wird (bei physischer Anbringung oder Projektion) oder nachträglich in jedes Bild als Bildinformation eingefügt wird und somit in den digitalen Daten von Bildern wieder gefunden werden kann. Die Identifikation eines Patienten kann neben einer solchen optischen Information, die z.B. drucktechnisch auf einem Identifikator angebracht sein kann, auch durch den Identifikator selbst erfolgen, beispielsweise durch dessen physische oder auch projizierte Formgestaltung.

Es kann sich hierbei in einem einfachsten Fall um die äußere geometrische Form eines Identifikators handeln.

Physische Identifikatoren können z.B. als Klebeetiketten ausgebildet und z.B. auf einem Haftgrund lösbar angeordnet sein und, wie zuvor benannt, durch ihre Formgestaltung einen zugeordneten Patienten identifizieren oder aber dies durch eine auf dem Klebeetikett getragenen optischen Information, die z.B. aufgedruckt sein kann, erreichen.

In einer besonders einfachen Ausführung kann somit beispielsweise ein Satz von Identifikatoren vorliegen, die alle eine bestimmte erste äußere geometrische Form haben und somit durch diese Form auf einen bestimmten Patienten hinweisen, also diesem zugeordnet sind, wohingegen in einem anderen Satz von Identifikatoren diese Identifikatoren eine andere äußere Formgestaltung aufweisen kann und somit einer anderen Person als Patient zugeordnet sein können oder zugeordnet werden. Ebenso besteht die Möglichkeit, wie eingangs erwähnt, eine optische Information auf Klebeetiketten beliebiger äußerer Form, also auch einer gegebenenfalls genormten oder einheitlichen Form aufzudrucken, wie z.B. eine Zahlenfolge, ein Barcode oder sonstige Art von optischer Information.

Soweit im Rahmen dieser Beschreibung von einer Zuordnung eines Identifikators zu einem Patienten gesprochen wird, kann dies dadurch erfolgen, dass dieser Identifikator, also beispielsweise anhand dessen Form oder anhand der von ihm getragenen optischen Information, dem Patientendatensatz eines Patienten zugeordnet wird.

Die digitalen Daten eines Bildes können sodann, nachdem diese auf das Vorhandensein eines Identifikators untersucht wurden und ein solcher Identifikator in den digitalen Daten festgestellt wurde, in den Patientendatensatz des durch den Identifikator bzw. dessen Information identifizierten Patienten übernommen werden. Diese digitalen Daten sind sodann mit dem Patientendatensatz verbunden und gespeichert und können zu jeder Zeit nachträglich abgerufen werden.

Dabei erfolgt bevorzugt diese Zuordnung und Übernahme automatisch, wofür es vorgesehen sein kann, dass das Untersuchen digitaler Daten auf das Vorhandensein wenigstens eines Identifikators mittels eines Archivierungssystems erfolgt, also z.B. einem solchen System, welches in Verbindung mit einer Patientendatenbank arbeitet und in welchem sämtliche Daten, bevorzugt zu allen Patienten eines Arztes, eines Krankenhauses, einer Klinik oder einer sonstigen untersuchenden Person, gespeichert sind. Hierfür kann es vorgesehen sein, dass innerhalb eines solchen Archivierungssystems eine Software arbeitet, welche die digitalen Daten auf das Vorhandensein solcher Identifikatoren untersucht und diese erkennt.

Demnach kann es sich hierbei um eine Bildverarbeitungs- und/oder Mustererkennungssoftware handeln, die in ein solches Archivierungssystem integriert sein kann.

Mit einem solchen Archivierungssystem bzw. einer solchen Software können somit erfindungsgemäß alle digitalen Daten aller Bilder, die in einem beliebigen Zeitraum auch von einer Vielzahl von Patienten gemacht wurden bearbeitet bzw. untersucht werden, wobei automatisch die richtige Zuordnung eines jeweiligen Bildes zu dem betreffenden Patienten erfolgt, da jedes Bild zumindest einen Identifikator in der Bildinformation aufweist, welcher den Patienten identifiziert.

In einer weiteren bevorzugten Ausführung kann es vorgesehen sein, dass es nicht nur einen Identifikator gibt, welcher den gerade fotografierten Patienten identifiziert, sondern auch solche Identifikatoren, die einem zu fotografierenden Areal, wie beispielsweise bestimmten Extremitäten, wie Hand, Fuß, Arme, Beine etc. eines Patienten und/oder Aufnahmebedingungen zugeordnet sind bzw. solche kennzeichnen.

Es kann auch vorgesehen sein, statt der eingangs genannten Möglichkeit, sowohl für die Identifikation des Patienten als auch für die Identifikation von Arealen oder Aufnahmebedingungen jeweils separate Identifikatoren vorzusehen, dass alle optischen Informationen (z.B. Patient, Areal, Aufnahmebedingung etc.), die an einem Areal vor dessen fotografischer Aufnahme angebracht werden, physisch durch denselben Identifikator, also z.B. Aufkleber / Klebetikett, Projektion, digitale Bildinformation oder auch einen Chip gebildet werden.

So kann ein solcher Identifikator nicht nur die Zuordnung zu einem bestimmten Patienten bewirken, sondern auch die Zuordnung zu einem Areal oder zu Aufnahmebedingungen, nämlich dadurch dass entsprechende Informationen zu diesen verschiedenen Möglichkeiten alle gleichzeitig auf einem solchen Identifikator, z.B. Aufkleber oder Chip angeordnet sind. So kann, beispielsweise in dem Fall, wenn ein solcher Identifikator als Aufkleber, Projektion oder digitale Daten ausgebildet ist, ein solcher Identifikator mehrere verschiedene optische Informationen umfassen, die jeweils eine der vorgenannten Informationen, d.h. die Identifikation von Patient, Areal oder Aufnahmebedingung repräsentieren oder aber auch eine gemeinsame optische Information, in der die vorgenannten mehreren Informationen gemeinsam geschlüsselt sind.

In einer möglichen Ausführung des erfindungsgemäßen Verfahrens kann es vorgesehen sein, dass die notwendigen optischen Informationen zur Durchführung der jeweiligen eingangs genannten Zuordnungen, d.h. zu Patient, Areal und gegebenenfalls Aufnahmebedingungen charakterisierenden Merkmalen, bereits an einem jeweiligen Identifikator ursprünglich angebracht sind, z.B. durch Aufdrucken, und einem Archivierungssystem so mitgeteilt werden, dass dieses die mitgeteilte Information einem ausgewählten Patienten oder Patientendatensatz zuordnet.

Alternativ dazu kann es ebenso vorgesehen sein, dass die optische Information in Abhängigkeit zumindest von einem Patienten bzw. Patientendatensatz und/oder vom zu fotografierenden Areal vom Archivierungssystem selbst erstellt wird und an wenigstens einem Identifikator angeordnet wird, beispielsweise durch Aufdrucken, durch Generieren einer Projektionsmaske oder Generieren von in ein Foto einzufügenden digitalen Bildinformationen.

So kann es z.B. in Verbindung mit den eingangs genannten Klebeetiketten vorgesehen sein, dass mit Hilfe des Archivierungssystems ein Satz von Klebeetiketten mit einem bestimmten Aufdruck erstellt wird, wobei dieser Aufdruck eine optische Information darstellt, die gemäß der Erfindung zumindest den Patienten identifiziert und gegebenenfalls bei unterschiedlichen Etiketten sodann auch unterschiedliche Areale. Bei der Projektionsanwendung kann es z.B. vorgesehen sein, dass eine programmierbare Projektionsmaske vorliegt, dessen Maskenprogrammierung von dem Archivierungssystem generiert wird. Z.B. kann eine solche Maske als durchleuchtbare LCD-Maske ausgebildet sind.

In einer bevorzugten Ausführung des Identifikatorsystems mit Bezug auf die zuvor genannten Ausführungsmöglichkeiten des Verfahrens kann es beispielsweise vorgesehen sein, dass die Klebeetiketten auf dem eingangs genannten Haftgrund in der geometrischen Form eines symbolisierten Lebewesens, insbesondere eines Menschen, angeordnet sind, insbesondere wobei ein Etikett, welches ein zu fotografierendes Areal des realen Lebewesens identifiziert, auf dem Haftgrund an der korrespondierenden Position des symbolisierten Lebewesens angeordnet ist.

Dies erleichtert die Arbeit einer fotografierenden oder videografierenden Person, da beispielsweise in dem Fall, wenn ein Hautareal an einer Hand aufgenommen werden soll, die aufnehmende Person ein Klebeetikett vom Haftgrund des Identifikatorsystems abzieht, welches an der Handposition des symbolisierten Menschen auf dem Haftgrund angeordnet ist.

Ein derartiges Klebeetikett weist demnach eine optische Information auf, welche neben dem Patienten auch das aufzunehmende Areal, in diesem Beispiel der Hand, kennzeichnet. Ein solches Etikett wird demnach bei der konkret zu untersuchenden, realen Person auf die Hand bzw. das jeweils betroffene Areal aufgeklebt und zusammen mit dem Hautareal im Bild erfasst. Hierbei kann es wie eingangs genannt auch bei dem Identifikatorsystem mit dieser vorgenannten symbolisierten Anordnung der Klebeetiketten vorgesehen sein, dass diese entweder vorab bedruckt sind mit entsprechenden optischen Informationen und diese Informationen dem Archivierungssystem mitgeteilt werden oder umgekehrt das Archivierungssystem die optischen Informationen aufdruckt.

Neben der Möglichkeit, dass ein Satz von Identifikatoren gebildet wird durch mehrere, von einem Haftgrund abziehbare Etiketten, die alle zumindest eine einen Patienten identifizierende optische Information aufweisen und/oder eine das Areal identifizierende Information kann es auch vorgesehen sein, dass separate Identifikatoren eine Aufnahmebedingung und/oder Kalibrierungsinformation umfassen oder eine solche Kalibrierungsinformation ebenso gleichzeitig zu den vorgenannten Informationen auf einem einzigen Identifikator angeordnet ist, insbesondere wiederum als optische Information oder geometrische Form, wobei eine solche Kalibrierungsinformation z.B. dazu dienen kann, eine Information zur Kalibrierung der Belichtung und/oder räumlichen Ausrichtung zwischen Kamera und aufzunehmendem Areal bereitzustellen bzw. anhand dieser Information eine solche Kalibrierung durchzuführen.

Hierbei wird unter der Kalibrierung der Belichtung nicht nur eine solche Kalibrierung bezüglich Helligkeit, sondern beispielsweise auch bezüglich der Farbe verstanden. Beispielsweise kann eine farbliche Information als Kalibrierungsinformation auf einem solchen Identifikator vorgesehen sein, die mit einem Bild erfasst wird, so dass ein Nutzer an einem Computer die Möglichkeit hat, die in einem Bild dargestellte Kalibrierungsinformation auf einen bestimmten Helligkeits- oder Farbwert einzustellen, um hierdurch die gesamte fotografische Information zu kalibrieren bzw. zu eichen und somit beispielsweise Bilder zu demselben Areal, die an zeitlich verschiedenen Orten und somit auch verschiedenen Belichtungssituationen aufgenommen wurden, untereinander vergleichbar zu machen.

Ebenso kann es vorgesehen sein, dass ein Identifikator als ein zwei-oder auch dreidimensionaler Körper, ggfs. ebenso klebender Körper ausgebildet ist oder eine zwei- oder dreidimensionale Information trägt und dass aus dessen Abbildung in dem Bild nicht nur identifizierende optische Informationen gewonnen werden sondern auch Informationen geometrischer Art z.B. über die Perspektive bei der Aufnahme oder sonstige räumliche Informationen. Z.B. erscheint ein rundes Plättchen oder ein dargestellter Kreis elliptisch, wenn es/er schräg aufgenommen wurde. So kann die gesamte Bildinformation anhand eines solchen Identifikators bildlich normiert werden auf eine skalierbare Standardansicht, z.B. immer senkrecht von oben, selbst wenn die fotografische Aufnahme tatsächlich nicht so aufgenommen wurde.

Ein erfindungsgemäßes Identifikatorsystem kann nicht nur physisch existieren, sondern auch virtuell gegeben sein als digitale Daten, insbesondere Musterdaten, die in ein Bild eines aufzunehmenden Areals eingefügt werden. Dies kann automatisch, z.B. durch eine Kamera erfolgen oder auch manuell durch einen Benutzer. Z.B. können die digitalen Daten auf einem Speicher vorliegen und aus diesem bei oder auch nach der Aufnahme in die Bilder eingefügt werden. Ein solcher Speicher kann Teil einer Kamera sein oder auch separat vorliegen, z.B. in der Art eines Memory-Sticks.

Bei der Ausführung mit projizierbaren Indentifikatoren kann eine Projektionseinheit vorgesehen sein, mittels der Identifikatoren auf das zu fotografierende Areal eines Patienten projiziert werden können. Ein solche Projektionseinheit kann in einer möglichen Ausführung auch direkt in einer Kamera angeordnet sein, mit der die Aufnahmen gemacht werden.

Neben der Bilddokumentation z.B. mittels der Erfassung von Fotos kann die Erfindung auch eingesetzt werden zur Bilddokumentation mittels Erfassung von Videos, also Bildsequenzen. So können z.B. auch Operationen gefilmt werden und die Bilder durch Kennzeichnung des Patienten jederzeit nachträglich genau einem Patienten zugeordnet werden.

Allgemein ist hier unter dem Begriff eines Patienten jede Person gemeint, von der ein Bilddokumentation gemacht werden soll, ohne Beschränkung auf den medizinischen Bereich. Insbesondere sind Anwendungen der Bilddokumentation im Bereich der Kosmetik ausdrücklich von der Erfindung umfasst.

Ein Ausführungsbeispiel der Erfindung ist in der nachfolgenden Figur symbolisch dargestellt.

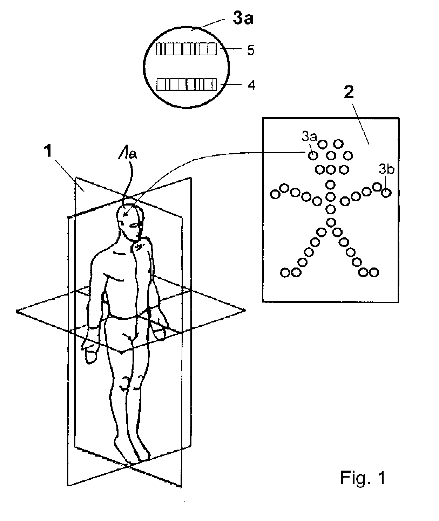

Die Figur 1 zeigt hier symbolisch dargestellt eine Person 1 als Patient, bei der verschiedene Hautareale des Körpers fotografisch dokumentiert werden sollen. Hierfür steht dem Arzt gemäß dieser hier beschriebenen Ausführung ein Identifikatorsystem 2 zur Verfügung, welches ausgebildet ist als eine Vielzahl von Etiketten 3a, 3b, ...., die auf einem Haftgrund angeordnet sind in der symbolischen Form eines Menschen mit seinen Extremitäten.

So ist hier erkennbar, dass ein Etikett mit dem Bezugszeichen 3a einem Kopf und mit dem Bezugszeichen 3b, z.B. einer linken Hand, zugeordnet ist, so dass sich hier eine Vielzahl von Etiketten in diesem Identifikatorsystem ergibt, die jeweils unterschiedlichen, jedoch genau definierten Arealen am Körper eines Menschen zugeordnet sind und dieses kennzeichnen.

Möchte nun, wie in diesem Beispiel dargestellt, ein Arzt eine fotografische Aufnahme der Haut am Kopf eines Patienten aufnehmen, so entnimmt der untersuchende Arzt das Etikett 3a vom Identifikatorsystem und klebt dieses auf die betreffende Stelle am Kopf des Patienten 1. Sodann wird vom Kopf bzw. dem interessierenden Hautareal eine fotografische Aufnahme gemacht, in der gleichzeitig auch das aufgeklebte Etikett 3a mitfotografiert wird. Dabei zeigt die Detaildarstellung einen beispielhaften Aufbau eines solchen Klebeetiketts 3a, welches einen Aufdruck sowohl als Patientenidentifikation 4 in Form eines Barcodes als auch einen weiteren Aufdruck in Form eines Barcodes 5 zur Identifikation des jeweiligen Areals umfasst.

Die Patientenidentifikation 4, d.h. hier der Barcode als optische Information, ist dabei auf allen Etiketten des hier dargestellten Identifikatorsystems 2 identisch, wohingegen die Arealidentifikation 5 auf dem jeweiligen Etikett je nach Areal unterschiedlich ist.

Aus dem aufgenommenen Foto, welches ein Hautareal des Kopfes zusammen mit dem Etikett 3a zeigt, kann nachträglich im Anschluss an eine Aufnahme, gegebenenfalls auch Tage später, mittels einer Mustererkennungssoftware das Etikett im Foto erkannt und die beiden Barcodes ausgelesen werden, um sodann zum einen dieses Foto dem richtigen Patienten zuzuordnen und gleichzeitig mit diesem Foto eine Information zu speichern, zu welchem Areal dieses Foto gehört.

Bezüglich sämtlicher Ausführungen ist festzustellen, dass die in Verbindung mit einer Ausführung genannten technischen Merkmale nicht nur bei der spezifischen Ausführung eingesetzt werden können, sondern auch bei den jeweils anderen Ausführungen. Sämtliche offenbarten technischen Merkmale dieser Erfindungsbeschreibung sind als erfindungswesentlich einzustufen und beliebig miteinander kombinierbar oder in Alleinstellung einsetzbar.

## Patentansprüche

1. Verfahren zur Zuordnung von Bilddokumenten zu einem Patienten, **dadurch gekennzeichnet, dass**
a. wenigstens ein bildlich aufzunehmendes Areal (1 a) wenigstens eines Patienten (1) mit wenigstens einem Identifikator (3a) **gekennzeichnet** wird,
b. wobei jedes aufzunehmende Areal (1a) wenigstens einen Identifikator (3a) beinhaltet, der einem Patienten (1) zugeordnet ist oder in einem späteren Schritt zugeordnet wird
c. mit wenigstens einem Identifikator (3a) **gekennzeichnete** Areale (1a) jeweils zusammen mit dem wenigstens einen Identifikator (3a) als Bild oder Film, insbesondere Digitalfoto oder Digitalvideo erfasst werden oder in das Bild eines Areals (1a) nachträglich wenigstens ein Identifikator (3a) eingefügt wird;
d. jedes Bild digitale Daten darstellt oder in digitale Daten gewandelt wird,
e. die digitalen Daten wenigstens eines Bildes, insbesondere eines Datensatzes von mehreren Bildern, insbesondere verschiedener Patienten (1) untersucht werden auf das Vorhandensein wenigstens eines Identifikators (3a)
f. ein vorhandener Identifikator (3a) aus den digitalen Daten ausgelesen wird und anhand des so gewonnenen wenigstens einen Identifikators das Bild und/oder die digitalen Daten der Bilder dem durch den Identifikator identifizierten Patienten (1) zugeordnet wird.

2. Verfahren nach Anspruch 1, **dadurch gekennzeichnet, dass** die Zuordnung eines Identifikators (3a) zu einem Patienten (1) so erfolgt, dass dieser Identifikator (3a) einem Patientendatensatz eindeutig zugeordnet wird, insbesondere wobei die digitalen Daten eines Bildes, welche einem Patienten (1) zugeordnet werden, in den Patientendatensatz dieses Patienten übertragen werden.

3. Verfahren nach einem der vorherigen Ansprüche, **dadurch gekennzeichnet, dass** das Untersuchen digitaler Daten auf das Vorhandensein wenigstens eines Identifikators (3a) mittels eines Archivierungssystems erfolgt, in welchem Patientendaten gespeichert sind, insbesondere mittels einer Bildverarbeitungs- und/oder Mustererkennungssoftware.

4. Verfahren nach einem der vorherigen Ansprüche, **dadurch gekennzeichnet, dass** ein Identifikator (3a) das aufzunehmende Areal (1a) und/oder Aufnahmebedingungen kennzeichnet bzw. diesem/diesen zugeordnet ist, insbesondere wobei alle Identifikatoren (3a) eines Areals (1a) physisch durch denselben Identifikator, insbesondere Aufkleber oder Chip gebildet werden.

5. Verfahren nach einem der vorherigen Ansprüche, **dadurch gekennzeichnet, dass** die optische Information (4, 5) wenigstens eines Identifikators (3a) einem Archivierungssystem mitgeteilt wird und dieses die mitgeteilte Information einem Patienten oder Patientendatensatz zuordnet, insbesondere bevor die automatisierte Untersuchung und Übernahme der vermittels dieses Identifikators aufgenommener digitaler Daten startet oder die optische Information (4, 5) in Abhängigkeit zumindest von einem Patienten (1) / Patientendatensatz und/oder vom aufzunehmenden Areal (1a) vom Archivierungsystem erstellt und an wenigstens einem Identifikator (3a) angeordnet wird, insbesondere durch Aufdrucken.

6. Verfahren nach einem der vorherigen Ansprüche, **dadurch gekennzeichnet, dass** ein Identifikator (3a) eine Kalibrierungsinformation umfasst, insbesondere durch eine am Identifikator (3a) angeordnete optische Information und/oder die geometrische Form des Identifikators (3a), insbesondere wobei die Kalibrierungsinformation eine Information zur Kalibrierung der Belichtung und/oder räumlichen Ausrichtung zwischen Kamera und aufzunehmenden Areal (1a) umfasst.

7. Verfahren nach einem der vorherigen Ansprüche, **dadurch gekennzeichnet, dass** ein Identifikator (3a) einem vorgefertigten Satz (2) von Identifikatoren (3a) entnommen wird, die alle demselben Patienten zugeordnet sind oder werden und jeweils unterschiedliche zu fotografierende Areale (1a) desselben Patienten (1) kennzeichnen.

8. Verfahren nach Anspruch 7, **dadurch gekennzeichnet, dass** ein Satz (2) von Identifikatoren (3a) gebildet wird durch mehrere von einem Haftgrund abziehbare Etiketten die alle wenigstens eine einen Patienten (1) identifizierende optische Information (4) aufweisen.

9. Verfahren nach Anspruch 8, **dadurch gekennzeichnet, dass** ein Identifikator (3a) zusätzlich zu der den Patienten (1) identifizierenden Information (4), wenigstens eine weitere Information (5) aufweist, insbesondere eine das Areal (1a) identifizierende Information und/oder Kalibrierungsinformationen aufweist, insbesondere wobei zumindest die Informationen (4, 5) hinsichtlich Patientenidentifizierung und Areal (1a) in derselben oder in separaten optischen Informationen (4, 5) enthalten sind.

10. Verfahren nach einem der vorherigen Ansprüche, **dadurch gekennzeichnet, dass** Identifikatoren (3a), die den Patienten (1) und/oder verschiedene Areale (1a) kennzeichnen, vermittels optischer Einrichtungen als Muster auf das zu dokumentierende Areal projiziert werden.

11. Verfahren nach Anspruch 10, **dadurch gekennzeichnet, dass** eine Kalibrierung und Skalierung der aufgenommenen Bilder aus der Verzerrung und Größe der projizierten Identifikatoren (3a) berechnet wird.

12. Verfahren nach einem der vorherigen Ansprüche, **dadurch gekennzeichnet, dass** Identifikatoren (3a), die den Patienten (1) und/oder verschiedene Areale (1 a) kennzeichnen, vor oder nach jeder Aufnahme als grafische Muster insbesondere von einem die betreffenden Identifikatoren enthaltenden Speicher, z.B. Memory-Stick aus in das zu archivierende Foto eingefügt werden.

13. Identifikatorsystem zur Zuordnung von Fotodokumenten zu einem Patienten **dadurch gekennzeichnet, dass** es einen Satz (2) von mehreren Identifikatoren (3a) aufweist, mit denen ein fotografisch oder videografisch aufzunehmendes Areal (1a) eines Patienten (1) kennzeichenbar ist, insbesondere durch Anbringung wenigstens eines Identifikators (3a) oder durch Projektion wenigstens eines Identifikators auf das zu dokumentierende Areal mittels optischer Einrichtungen, wobei jeder Identifikator (3a) mit wenigstens einer optischen Information (4) versehen oder versehbar ist, die zumindest einem Patienten (1) und/oder einem aufzunehmenden Areal zugeordnet ist oder zuordenbar ist.

14. System nach Anspruch 13, **dadurch gekennzeichnet, dass** die Identifikatoren (3a) als Klebeetiketten ausgebildet sind, die auf einem Haftgrund lösbar angeordnet sind, insbesondere mit wenigstens einer aufgedruckten optischen Information (4), insbesondere wobei die Klebeetiketten (3a) auf dem Haftgrund in der geometrischen Form eines symbolisierten Lebewesens, insbesondere eines Menschen angeordnet sind, insbesondere wobei ein Etikett (3a), welches ein aufzunehmendes Areal (1a) des realen Lebewesens identifiziert auf dem Haftgrund an der korrespondierenden Position des symbolisierten Lebewesens angeordnet ist.

15. System nach einem der vorherigen Ansprüche 13 oder 14, **dadurch gekennzeichnet, dass** eine Kalibrierung und Skalierung der aufgenommenen Bilder aus der Verzerrung und Größe der projizierten Identifikatoren (3a) berechenbar ist.
